# EUROPEAN PATENT APPLICATION

(11) **EP 4 702 944 A1**
(43) Date of publication of application: **04.03.2026**
(21) Application number: 24197286.8
(22) Date of filing: 29.08.2024
(51) Int. Cl.: A61B 34/10, A61C 7/00

(54) **MEANS FOR PLANNING AN IMPLANTATION OF A DENTAL IMPLANT**

(71) Applicant: Visiogenics UG (haftungsbeschränkt), 60486 Frankfurt am Main (DE)
(72) Inventor: GHANAATI, Shahram, 60486 Frankfurt am Main (DE); SADER, Robert, 60528 Frankfurt am Main (DE); GHANAATI, Amir, 60528 Frankfurt am Main (DE); HESELICH, Anja, 63743 Aschaffenburg (DE)
(74) Representative: Herrmann, Daniel

(57) **Abstract**

Described are means for planning of an implantation of a dental implant. The means include a method of planning an implantation of a dental implant, the method comprising: receiving one or more radiological images, the received radiological images comprising at least a first radiological image of a region of a jaw in which region a tooth extraction socket is located, wherein the first radiological image was taken at a first point in time, which first point in time being after an extraction of a tooth from the region of the jaw; analysing the one or more received radiological images including analysing the first radiological image; generating an implantation plan for the implantation of the dental implant in the region of the jaw based on the analysed radiological images, the generating comprising: determining a position for the dental implant in the region of the jaw, which position indicates a probable position of the tooth before its extraction in the region of the jaw; and indicating the determined position in the implantation plan; and providing the generated implantation plan.

## Description

### TECHNICAL FIELD

The present invention relates to a method for planning an implantation of a dental implant, a corresponding storage medium, a corresponding computer programme, a corresponding computing system or device and an implantation aid to assist in positioning a dental implant.

### BACKGROUND

When a tooth is extracted from the human jaw, the resulting extraction socket is typically left to heal before a dental implant is implanted to replace the extracted tooth. The loss of the tooth usually leads to jaw bone atrophy, especially in jaw bone ridge height and buco-lingual width of the jaw bone at the extraction site. This is due to reduced mechanical interaction of the jaw at the position of the extracted tooth, which interaction provides biological stimuli to keep the jaw bone intact.

The dental implant, which replaces the extracted tooth is implanted according to an implantation plan, which specifies details about the implantation, such as e.g. the position at which the dental implant is to be implanted and the size of the dental implant to be implanted. According to the state of the art, the dental implant is determined to be placed in the remaining jaw bone in the region of the extraction site and its position is determined on the basis of the remaining jaw bone and the dimensions thereof (the dental implant is typically placed bucco-lingually centrally on the remaining jaw bone), as are further parameters, such as the size of the dental implant (typically based on the remaining bucco-lingual jaw bone width).

In recent years, complications in implantology that have led to implant loosening or loss, or subsequent infections (peri-implantitis) leading to potential jaw bone loss have been increasingly documented. In such cases implant removal is in some cases the only treatment option, placing a large burden on patients as well as medical practitioners.

### SUMMARY

It is thus an object of the present invention to provide a plan for an implantation of a dental implant, which plan decreases the probability of complications after the dental implant has been implanted.

This object is achieved by a method as defined by independent claim 1, by a storage medium as defined by claim 13, a computer programme as defined by claim 14, a computing system or device as defined by claim 15 and by an implantation plan as defined by claim 16.

The inventors have recognized that the position of the dental implant plays a crucial role in preventing post-operative complications because the implantation of dental implants in the remaining jaw bone primarily or solely on the basis of the remaining jaw bone and the dimensions thereof leads to increased mechanical stress placed on the dental implant. Due to frequently occurring displacements of the dental implant (as a consequence of the determined positions) in comparison to the position of the extracted tooth before its extraction, the dental implant does not fit into the biomechanical structure of the jaw and the remaining teeth. The inventors have further realised that the increased mechanical stress promotes loosening of the dental implant in the jaw bone, which then may lead to subsequent infections. The inventors have further realised that the placement of the dental implant is relevant to the occurrence of other subsequent illnesses. When the dental implant is placed such that irritations of other oral structures, such as the tongue, occur, these irritations may, with prolonged exposure cause irritations in the other oral structures. These irritations may lead to any number of subsequent pathologies, e.g. inflammation, cancerous growths etc. The inventors have developed a method by which the probability of such post-operative complications is decreased.

According to a general aspect of the invention, a method for planning an implantation of a dental implant is provided, the method comprising: receiving one or more radiological images, the received radiological images comprising at least a first radiological image of a region of a jaw in which region a tooth extraction socket is located, wherein the first radiological image was taken at a first point in time, which first point in time being after an extraction of a tooth from the region of the jaw; analysing the one or more received radiological images including analysing the first radiological image (e.g. analysing a jaw bone structural feature present in the first image); generating an implantation plan for the implantation of the dental implant in the region of the jaw based on the analysed radiological images, the generating comprising: determining a position for the dental implant in the region of the jaw, which position indicates a probable position of the tooth before its extraction in the region of the jaw; and indicating the determined position in the implantation plan; and providing the generated implantation plan.

By the above method involving analysis of the first radiological image, the position for the dental implant is determined such that it corresponds to the probable position of the tooth before its extraction, in contrast to conventional practice, in which the (probable) position of the tooth before its extraction in the region of the jaw is not taken into account for the determination of the position for the dental implant. In this context, the analysis of the first radiological image provides are particularly effective way (e.g. analysing a jaw bone structural feature present in the first image) of determining the probable position of the tooth before its extraction in the region of the jaw. The position of the original tooth provides a biomechanically stable and healthy position for the dental implant, in which position the mechanical stress on the dental implant after implantation is much reduced, compared to other suboptimal positions. Due to the reduced mechanical stress on the implanted dental implant, loosening of the dental implant after implantation is much reduced. Thus, additional medical procedures due to periimplantitis or other consequential illnesses as described above may frequently be avoided. Moreover, fewer or less prominent physical obstacles are introduced into the patient's mouth, since the dental implant is positioned in the probable position of the tooth before its extraction, which tooth and its position the patient is used to and to which position other oral structures are adapted. Therefore, long-term adverse effects, such as inflammation of other oral structures, such as the tongue, and e.g. cancerous growths due to such inflammation are further reduced.

In another aspect of the invention, the first point in time is after a period of time for a partial or complete jaw bone regeneration phase in the region of the jaw after the extraction of the tooth. By using the received one or more images, which comprise at least the first radiological image, which first radiological image was taken first point in time, which first point in time being after an extraction of a tooth from the region of the jaw and after a period of time for a partial or complete jaw bone regeneration phase, data reflecting a state at which the extraction socket was allowed to heal more thoroughly is taken into account when planning the dental implant, thus reducing the risk of post-operative inflammation and/or infection.

According to another aspect of the invention, the first point in time may be before a start of a jaw bone regeneration phase in the region of the jaw. Alternatively or additionally, the first point in time may be directly after the extraction of the tooth in the region of the jaw, preferably less than one week after the extraction, more preferably, less than three days after the extraction, more preferably less than twenty four hours after the extraction, more preferably after less than twelve hours after the extraction, more preferably after less than three hours after the extraction. By the use of the first radiological image, which image may be taken before a start of a jaw bone regeneration phase in the region of the jaw, such as directly after the extraction of the tooth, the probable position of the tooth before its extraction may be determined more easily based on the extraction socket, which at this point in time has typically not yet healed, such that it may indicate with high certainty the probable position of the tooth, and thus, the position for the dental implant.

According to another aspect of the invention, the received radiological images further comprise a second radiological image of the region of the jaw, wherein the second radiological image was taken at a second point in time, which second point in time being before the first point in time and before the period of time for the jaw bone regeneration phase.

The inclusion of the second radiological image, taken at a second point in time, which second point in time being before the period of time for the jaw bone regeneration phase, allows a more accurate determination of the probable position of the tooth before its extraction in the region of the jaw. This benefit is achieved because before the jaw bone regeneration phase the extraction socket has not yet healed, such that an indication of the probable location of the tooth before the extraction exists in said image. Furthermore, atrophy of the jaw bone in the region of the jaw bone is not yet fully realized, such that the dimensions of the jaw bone in the region of the jaw bone provide further basis for the determination of the probable position of the tutor for extraction.

According to another aspect of the invention, the received radiological images further comprise a second radiological image of the region of the jaw, wherein the second radiological image was taken at a third point in time, which third point in time being after the first point in time and preferably after a period of time for the partial or complete jaw bone regeneration phase.

The use of a second radiological image, taken at the second or third point in time, is beneficial due to the possibility of observing changes between the points in time directly and without the need for further investigation other than the direct comparison between the images, such as may be done in any number of ways, e.g. by superimposing sectional images of the radiological images, as is described in further detail below. Thus, when the first point in time is directly after the extraction of the tooth in the region of the jaw, it is beneficial for said reasons for the second radiological image to be taken at said third point in time.

In a preferred aspect of the invention, the first point in time and the second/third point in time are separated by at least four weeks. However, other time spans are equally applicable, such as eight weeks, three months, for months, five months, six months, one year, two years etc. Such time spans provide enough time for socket healing to begin, which is beneficial for the implantation of a dental implant.

In a further preferred aspect of the invention, the received radiological images are three-dimensional images, preferably three-dimensional computed tomography images, preferably CBCT-images, more preferably denoised CBCT-images.

Three-dimensional images allow a holistic approach to the planning of a dental implant, where all relevant information may be extracted from the image data. Computed tomography provides high resolution images, which are beneficial for the method of the present invention, since small inaccuracies can lead to the dental implant not fitting correctly or not being securely placed in the position for the dental implant. CBCT imaging devices are ubiquitous in dental practices globally, such that the use of CBCT-images is particularly beneficial, since this allows a large number of patients and medical practitioners to benefit from the improved implantation plans. As described above, inaccuracies, where possible, be avoided, such that the received radiological images should preferably be denoised.

According to a further aspect of the invention, the analysing of the one or more received radiological images further comprises: generating sets of sectional images of the region of the jaw from the one or more received radiological images, wherein every set contains at least one sectional image of at least some of the received images, wherein the sectional images in every set correspond to one another, and wherein the sectional images in every set preferably have the same sectional plane and orientation. In a preferred aspect the sectional images of every set of sectional images are superimposed.

The generation of sets of sectional images provides further benefits to the above-mentioned holistic approach to the planning of the implantation of a dental implant, since the internal state of the jaw bone may better be assessed. It is particularly beneficial when the sectional images in every set correspond to one another and preferably, have the same sectional plane orientation, such that for every set of sectional images the same structures are depicted in the same way. This makes it possible for sectional images in a set of sectional images to be superimposed with each other. In a preferred aspect of the invention every set of sectional images contains at least one sectional image of the first received radiological image. In a further preferred aspect of the invention, every set of sectional images further contains at least one sectional image of the second received radiological image. Additionally, the superposition of sectional images allows a better determination of the probable position of the tooth before it extraction in the region of the jaw.

The inventors have further realized that during the healing process, cavitation formations may form in the jaw bone, when the bony wound heals faster than the extraction socket can be filled by bone regeneration. The inventors have understood that, contrary to common belief, the extraction socket persisting in the jaw bone after the extraction of the tooth in the region of the jaw, heals inward from the edges, leading to said cavitation formations. Such cavitation formation can be beneficial to the determination of the position for the dental implant, but may also impede the proper functioning of an implanted dental implant and may further increase the risk of infection. When a dental implant is implanted without heed to possible cavitation formations, the dental implant may not be rooted in the jaw bone as firmly as possible. Thus, when mechanical stress is administered to the dental implant, for example, when the patient is chewing or grinding their teeth, and resolved cavitation formations may increase the risk of the dental implant loosening. Such loose dental implants provide a source of infection and discomfort to patients, thus requiring further treatment. Moreover, empty cavitation formations may themselves increase the risk of infection even without the dental implant being drilled into the cavitation formation and thus loosening the dental implant. Since the extraction socket heals inwards from the edges, thereby creating such cavitation formations, said formations may be used in the determination of the (probable) position of the tooth before its extraction. The inventors have further understood that when using a first and second radiological image taken at different points in time, which points in time being separated by the jaw bone regeneration phase, cavitation formations are provided a period of time in order to resolve by themselves whilst still providing the necessary information for the determination of the (probable) position of the tooth before its extraction.

According to another aspect of the invention, the analysing of the one or more received radiological images further comprises: identifying structural features and/or structural changes in the region of the jaw, particularly neighbouring structures of the tooth before its extraction from the region of the jaw, preferably neighbouring teeth, an extraction socket, a form of the jaw bone, cavitation formations and/or atrophies in the region of the jaw. Through the identification of structural features, such as a jaw ridge width, cavitation formations, distances between neighbouring oral structures etc. the implantation of a dental implant can be planned more effectively and in a more detailed manner, since such structural features directly pertain to aspects such as the position for the dental implant, the size of the dental implant to be implanted, the drill angle for the implantation of the dental implant, further operations which are necessary before or during the implantation, such as bone augmentation or cavitation resolution etc. Structural changes in the region of the jaw may pertain to atrophy of the jaw bone in the region of the jaw, the amount of which may be relevant for the determination of further operations before or during the implantation of the dental implant, such as jaw bone augmentation (e.g. according to the principle of guided open wound healing).

In a preferred aspect of the invention, the identification of structural features and/or structural changes is done based on the sets of sectional images, wherein the sets preferably each contain sectional images of the first radiological image. Preferably, the sets of sectional images each contain sectional images of the first and second radiological images. More preferably the identifying is done based on the superimposed sectional images of the first and second radiological images. The use of sectional images is particularly well-suited to the identification of cavitation formations, since the cavitation formations occur within the jaw bone. Since cavitation formations can resolve themselves, it is particularly useful to use sectional images of the first radiological image, taken after the jaw bone regeneration phase in the region of the jaw. In order to identify structural changes in the region of the jaw it is, furthermore, useful to use superimposed sectional images of the first and second radiological images, which are taken at two different points in time, which points in time are separated by said jaw bone regeneration phase.

According to a preferred aspect of the invention, the determining of the position for the dental implant in the region of the jaw is done based on the identified structural features and/or structural changes in the region of the jaw, preferably identified cavitation formations.

Structural features and/or structural changes in the region of the jaw occur or may develop during the healing process of the extraction socket. Since the extraction socket indicates the position of the tooth before its extraction in the region of the jaw, the structural features/changes in said region may themselves indicate said position with a high probability. For example, a cavitation formation may indicate a (probable) position of the tooth before its extraction, since, as described above, the extraction socket heals in a manner which favours the development of such cavitation formations at the position of the extraction socket. Thus, when such a cavitation formation is identified, it provides a basis for determination of said (probable) position of the tooth, which position may then be provided as the position for the dental implant. As another example, jaw bone atrophy may occur in the region of the jaw from which the tooth was extracted. Due to decreasing mechanical stimulation at the position of the extracted tooth, bony tissue may be broken down, as it is no longer required by the body to stabilise the tooth. As such, bone atrophy, such as jaw bone height loss (e.g. jaw ridge height loss) and jaw bone width loss (e.g. jaw ridge width loss) provide further indicators of the probable position of the tooth before its extraction, which may, thus, provide a basis for the determination position for the dental implant, which position being the probable position of the tooth before its extraction. As yet another example, the jaw bone form may be interpolated as described further in later paragraphs in order to determine the position for the dental implant, which position being the probable position of the tooth before its extraction. As yet another example, neighbouring structures may provide indications regarding the probable position of the tooth before its extraction, e.g. through the distance between the neighbouring teeth, the connection line of neighbouring teeth, etc.

In a further aspect of the invention, the identified structural changes comprise changes in a height and/or a width of a jaw ridge of the jaw in the region of the jaw.

According to another aspect of the invention generating the sets of sectional images further comprises: generating sectional images of the region of the jaw in occlusal, buccal, lingual, mesial and/or distal direction. By creating sectional images in distal and/or mesial direction the distance between neighbouring oral structures of the tooth before its extraction, such as neighbouring teeth, can be analysed, which allows for a better determination of details of the implantation plan such as the probable position of the tooth before its extraction in the region of the jaw, the size of the dental implant to be implanted in the region of the jaw etc. Sectional images in buccal and/or lingual direction, may aid in the determination of the jaw ridge width, which can be relevant for the decision of whether jaw bone augmentation is necessary before or during the implantation of the dental implant.

In a preferred aspect of the invention, the identifying of the change of a height of the jaw ridge is done on the basis of a reference line, which reference line preferably being a mesio-distal bone line in the sectional images of one of the received radiological images, wherein the mesio-distal bone line is a line connecting the jaw ridge at neighbouring oral structures of the tooth before its extraction in the region of the jaw, particularly between neighbouring teeth. Since the height of the jaw ridge at neighbouring all structures of the tooth before it extraction typically is not affected by the extraction of the tooth, the height of the jaw ridge at these structures may be used as a reference for the change of the height of the jaw ridge at the extraction site before the extraction. Thus, the change of the height of the jaw ridge may be found out by use of said reference. The mesio-distal bone line provides a direct and easy to implement tool for the determination of such a reference height. This mesio-distal bone line maybe identified most easily in the sectional images of the received radiological images, especially the sectional images in the mesial and/or distal directions. In order to provide the most accurate implantation plan it is beneficial for the mesio-distal bone line to be used as a reference line to be identified in the first radiological image, especially the sectional images of the first radiological image.

In a further preferred aspect of the invention, the identifying of the change of a height of a jaw ridge further comprises: determining an actual height of a jaw ridge in the region of the jaw by determining a tangent to the jaw ridge at the lowest point of the jaw ridge in the first radiological image and/or the sectional images of the first radiological image, the tangent being parallel to the reference line; and determining the distance between the tangent and the reference line.

In a particularly preferred aspect of the invention, the generating of the implantation plan further comprises: determining an implant type for the dental implant based on the distance between the tangent and the reference line. Dental implants have different types, such as tissue level or bone level implants, which differ in the construction by the amount of constituent elements (tissue level implants being made of one single element, whereas bone level implants consist of two elements). Furthermore, different implant types may be useful in different situations. For example, typically bone level implants are preferred when the aim is to set the implant in a region of the jaw, in which the implant can be "hidden" under the existing bone level in that particular region of the jaw. Tissue level implants without any additional protection by bone substitute mateirals showed to becomexposed under the soft tissue over time. Thus, presently, they are no more in favour of the majority of dentists.

According to another aspect of the invention, the analysing of the one or more radiological images further comprises: determining a mesio-distal distance between neighbouring oral structures of the tooth, particularly between neighbouring teeth in at least one of the received radiological images, preferably in the first radiological image and/or the sectional images of the first radiological image.

The mesio-distal distance between neighbouring oral structures of the tooth is particularly relevant for the proper determination of the position for the dental implant in the region of the jaw, since the position for the dental implant is constrained by the neighbouring oral structures.

Furthermore, a bucco-lingual width of the jaw ridge may be determined in at least one of the received radiological images, preferably in the first radiological image and/or the sectional images of the first radiological image. Moreover, the probable position of the tooth before its extraction may be determined from the second received radiological image and/or the sectional images of the second radiological image. The exact form of the jaw ridge and changes thereof may be determined from the first and second radiological images, especially from the superposition of sectional images of the first and second radiological images (preferably in buccal or lingual direction). When the probable position is determined in such a manner, the determination is typically more exact, since the extraction socket is not yet healed and may, thus, aid in the determination.

In a further aspect of the invention, the analysing of the one or more radiological images further comprises: determining a drill angle for a hole to be drilled for the implantation of the dental implant. The drill angle for the hole in which the dental implant will be implanted ensures that the mechanical stress applied to the dental implant closely matches the mechanical stress to which the tooth was exposed before it extraction, such that proper healing and firm stabilization of the jawbone around the implant can ensue. Furthermore, as described above, the mechanical stress applied to the dental implant and thereby transmitted to the jaw bone in the region of the jaw stimulate biological jaw bone reservation, such that a close match between the determined drill angle and the route of the extracted tooth is particularly beneficial.

According to another aspect of the invention, the generating of the implantation plan further comprises: determining a diameter of the dental implant based on the mesio-distal distance.

It is particularly beneficial the diameter of the dental implant to be implanted in the region of the jaw to be determined on the basis of the mesio-distal distance, rather than the remaining bucco-lingual width of the jaw ridge in the region of the jaw. This is due to the possibility of jaw bone argumentation, such that the bucco-lingual jaw ridge width becomes less relevant. The mesio-distal distance between neighbouring oral structures of the tooth, particularly between neighbouring teeth, thus, provides an indicator for the upper limitation for the diameter of the dental implant, whilst the bucco-lingual jaw ridge width may serve as an indicator of whether argumentation of the jaw bone in the region of the jaw is necessary for the implantation of the dental implant. For example, it may be determined, that based on the position for the dental implant, a distance of at least 0.5 mm, 1 mm, 1.5 mm, 2 mm or 2.5 mm must remain between the dental implant once implanted at the position for the dental implant and neighbouring teeth, thus limiting the diameter of the dental implant. It may further be specified, that the remaining distance between the dental implant once implanted at the position for the dental implant and a neighbouring dental implant (which may be an example of another neighbouring structure) must be at least 1.5 mm, 2 mm, 2.5 mm, 3 mm, 3.5 mm or 4 mm. The minimum distance between neighbouring structures of any kind may also be any plausible amount of distance.

In a preferred aspect of the invention, the determined position for the dental implant in the region of the jaw is determined as a set of three-dimensional coordinates in a reference coordinate system of one of the received radiological images.

The determination of the position in terms of a set of three-dimensional coordinates in a reference coordinate system of one of the radiological images provides a particularly exact determination of the position in line with the resolution of the received radiological images.

According to another aspect of the invention, the generating of the implantation plan further comprises: determining whether the implantation of the dental implant in the region of the jaw requires bone augmentation in the region of the jaw prior to and/or during the implantation. As described above, argumentation of the jaw bone provides the possibility for inducing bone growth in the region of the jaw, such that the width and/or the height of the jaw ridge may be increased. When the probable position of the tooth before its extraction is particularly close to the edge of the jaw ridge or even extends beyond the existing jaw bone in the region of the jaw (particularly in a buccal and/or a lingual direction), proper implantation can only occur at the desired position, when jaw bone argumentation is performed, the need for which must be determined prior to the implantation. In a particularly preferred aspect of the invention the generating of the implantation plan further comprises: determining how much bone augmentation in the region of the jaw is required prior to and/or during the implantation. Depending on the amount of atrophy which has occurred in the jaw bone in the region of the jaw different amount of augmentations may be necessary for the dental implant to be implanted. For example, it may be determined, that when there is no or very little change of the height of the jaw ridge (i.e. the distance between the reference line and the tangent is zero or close thereto, e.g. less than 1 mm, less than 0.5 mm, less than 0.1 mm or any other suitable boundary) no augmentation of the jaw bone is required. It may be determined that when the change of the height of the jaw ridge is significant, however is less or equal to .1.5 mm, 2mm, 3 mm, 4 mm, or any other suitable boundary the implantation requires augmentation, which can be performed during the implantation. It can be further specified, that when the change in the height lies within a range of 1.5 mm to 5 mm, 2 mm to 5 mm, 3 mm to 6 mm, 4 mm to 6 mm or any other suitable range, augmentation is required prior to implantation. Furthermore, it may be determined, that when the change of the height is more or equal to 4 mm, 5 mm, 6 mm, 7 mm or any other suitable boundary, augmentation is required prior to the implantation and may require more than one round of augmentation. This may be performed according to the guided open wound healing concept (Ghanaati et al.).

There further aspect of the invention, the generating of the implantation plan further comprises: determining whether existing cavitation formations in the region of the jaw must be resolved before and/or during the implantation of the dental implant. When cavitation formations exist in the region of the jaw they may pose problems for the implantation of the dental implant. For example when the hole to be drilled for the dental implant intersects the existing cavitation formations, this may hinder a firm stabilization of the dental implant in the jaw bone in the region of the jaw. Furthermore, cavitation formations may be inflamed and thus potentially lead to an inflammation or infection of the dental implant. Therefore it is beneficial to determine whether existing cavitation formations in the region of the jaw must be resolved before and/or during the implantation of the dental implant, such that consequential issues with the dental implant may be avoided.

According to an aspect of the invention, the providing of the implantation plan comprises: providing in the implantation plan information, which information specifies the determined type of dental implant. But the provision of said information the medical practitioner tasked with the implantation of the implant is enabled to choose the type of implant best suited to the case at hand.

In a further aspect of the invention, the providing of the implantation plan comprises: providing in the implantation plan information, which information specifies whether the implantation of the dental implant in the region of the jaw requires bone augmentation in the region of the jaw prior to the implantation. By making available the information regarding the required amount of bone augmentation in the implantation plan, better fitting dental implants (e.g. having the correct diameter etc.) may be chosen for implantation, such that the patient is exposed to less discomfort after the implantation. In a particularly preferred aspect of the invention, the providing of the implantation plan comprises: providing in the implantation plan information, which information specifies how much bone augmentation in the region of the jaw is required prior to the implantation.

In another aspect of the invention, the providing of the implantation plan comprises: providing in the implantation plan the determined diameter of the dental implant. By the provision of said diameter, medical practitioners are enabled to provide the best suited dental implant to a patient, thereby ensuring that further bone loss (atrophy) is decreased or even averted, thus, ensuring high-quality medical attention for the patient.

In a preferred aspect of the invention, wherein the providing of the implantation plan comprises: providing in the implantation plan the set of three-dimensional coordinates. The set of three-dimensional coordinate determines the position for the dental implant in the region of the jaw in a reference coordinate system of one of the radiological images. By providing the position for the dental implant in such a manner, the medical practitioner tasked with the implantation of the implant may view the position for the dental implant in a medical image viewer within one of the received radiological images. This allows for a detailed preparation of the implantation procedure for the medical practitioners. Preferably, the reference coordinate system is that of the first radiological image. This is particularly beneficial, since the first radiological image was taken after a jaw bone regeneration phase in the region of the jaw, such that the extraction socket is typically no longer a disturbing artefact in the radiological image. Furthermore, the position for the dental implant may be viewed in context of relevant structural features and/or changes.

According to an aspect of the invention, the providing of the implantation plan comprises: providing in the implantation plan information, which information specifies whether existing cavitation formations in the region of the jaw must be resolved before and/or during the implantation of the dental implant. This allows medical practitioners tasked with implementing the implantation plan for the implantation of the dental implant to take such actions such as the resolution of existing cavitation formations which otherwise could be detrimental to a successful implantation or the health of the patient after the implantation of the dental implant, for example due to developing information and/or infections.

According to a further aspect of the invention, the providing of the implantation plan comprises: providing in the implantation plan the determined drill angle. This enables those tasked with the implantation of the dental implant to perform the implantation, such that the dental implant is implanted into the region of the jaw in a manner which resembles most closely the setting of the tooth in the region of the job for its extraction in the region of the draft. This produces similar mechanical effects on the jaw bone, such that further consequential complications may be averted. Additionally, cavitation formations in the region of the jaw which are not inflamed and/or infected and which would not be intersected by the whole drilled for the dental implant when drilled correctly may be avoided when drilling by the medical practitioners, thereby further decreasing the risk of subsequent complications.

According to another aspect of the invention, the analysing includes: identifying a form of the jaw bone in the vicinity of neighbouring oral structures of the tooth before its extraction in the region of the jaw; and interpolating the identified form. Furthermore, the determining of the position for the dental implant in the region of the jaw is done based on the interpolated form.

Since changes in the jaw bone in the region of the jaw typically occur in the vicinity of the extraction site of the tooth before it extraction, other areas in the region of the jaw, such as in the vicinity of neighbouring oral structures of the tooth before it extraction, preferably neighbouring teeth, an interpolation of the form of the jaw bone from the identified form of the jaw bone in the vicinity of said neighbouring oral structures enables the determination of the probable position of the tooth before it extraction in images taken after the extraction of the tooth and even in images taken after a jaw bone regeneration phase. Thus, based on one received radiological image alone the probable position of the tooth before its extraction and thereby the position for the dental implant may be determined in a reliable manner.

The inventors have further realised, that the information specified in the provided implantation plan, particularly the position for the dental implant is not easily transferred to the actual implantation procedure. Oral surgery is particularly difficult due to the constrained space and small structures which must be surgically treated. Additionally, large forces must be administered, for example when drilling a hole for a dental implant. Thus, to further achieve the object of the invention, implantation aids may be produced with the help of the method of the invention or provided as part of the implantation plan, to aid medical practitioners in the implantation of dental implants according to the invitation plan derived by the method of this invention.

According to an aspect of the invention, the generating of the implantation plan further comprises: generating a file containing mesh coordinates of a three-dimensional data model of the dental implant, wherein the file is adapted to be used for additive manufacturing of an implantation aid according to the disclosure of this invention. When an implantation aid according to the disclosure of this invention is manufactured, details pertaining to the dental implants to be implanted, such as the diameter of the dental implant etc. are relevant to the manufacturing process. The file containing mesh coordinates of the three-dimensional data model of the dental implant contains the necessary specifications in order to adapt the manufacturing process, such that the invitation aid is adapted to the implantation of the modeled dental implant according to the implantation plan. In a preferred aspect of the invention, the providing of the implantation plan comprises: providing in the implantation plan the file. Thus, an implantation aid according to this disclosure may be manufactured independently of the actual implantation, for example by external manufacturers who themselves do not perform the implantation.

Generally, some of the operations recited herein in context of any one of the methods may be executed by one or more computers, while other operations may be executed manually, possibly with support of one or more computers. In an aspect of the invention, all the operations of the method of the invention, other than the provision of the implantation aid, may be performed by a computer. For example, the analysing of the images can be performed by a computer or manually with support by a computer. Image and data analysis may be performed by computers - or with support of computers - highly efficiently and with great precision, such that it is particularly beneficial for the analysis of the received radiological images.

Storage media storing instructions corresponding to the methods described herein, preferably of non-transitory kind, corresponding computer prorammes comprising corresponding commands and computing devices or systems adapted to perform the methods described herein can be provided.

In another aspect of the invention, an implantation plan for the implantation of a dental implant in a region of a jaw, which implantation plan is obtainable by the method of this disclosure, wherein the implantation plan entails a position, preferably three-dimensional coordinates, for the dental implant in a region of the jaw, which position indicates the probable position of a tooth before its extraction in the region of the jaw is provided. In a preferred aspect of the invention the implantation plan is used for the implantation of a dental implant in a region of a jaw. By using the implantation plan derived by the method of this disclosure for the implantation of a dental implant in the region of the jaw the dental implant can be implanted at the probable position of the tooth before it extraction, thereby decreasing the risk of consequential illnesses and/or complications. Furthermore the position for the dental implant in the region of the jaw is preferably provided in the form of three-dimensional coordinates, such that the position may be viewed within the context of radiological images of the region of the jaw. This allows a better operation of the implantation procedure.

According to another aspect of the invention, an .STL file, which file is obtainable by the method of this disclosure, wherein the file contains mesh coordinates of a three-dimensional data model of the dental implant to be implanted in a region of a jaw, wherein the file is adapted to be used for additive manufacturing of an implantation aid according to this disclosure is provided. By using and the data contained therein for the additive manufacturing of an implantation aid according to this disclosure, the implantation aid can be tailored to the dental implant to be implanted in the region of the jaw, thus, allowing more accurate implantation according to the implantation plan.

According to a further aspect of the invention, an implantation aid configured to be placed adjacent to an oral structure, preferably a tooth, to assist in positioning a dental implant in a region of a jaw at a position for the dental implant is provided. By being configured to be placed as described the implantation aid provides the medical practitioner tasked with the implantation of the dental implant support in positioning the dental implant correctly and according to the implantation plan.

In a preferred aspect of the invention, the implantation aid is configured to be placed on the structures, particularly teeth, surrounding the position for the dental implant. By said configuration the implantation aid may be placed in the direct surroundings of the position for the dental implant, such that the risk of illusions with the implantation aided during an implantation procedure outside of the region of the jaw are reduced. Furthermore by being configured to be placed on the structures, movements of the implantation aid relative to the structures can be further limited, thus increasing the accuracy with which the implantation aid may assist in positioning the dental implant. Additionally, the adaptation to being placed on the teeth surrounding the position of the dental implant, i.e. the neighbouring teeth, which are typically hard structures and are firmly rooted within the jaw bone, absolute movement of the implantation aid is further limited, thereby further increasing the accuracy.

According to another aspect of the invention, the implantation aid comprises a first aperture, the first aperture being designed such that when the implantation aid is correctly positioned in the region of the jaw, the first aperture indicates a drill angle for a hole to be drilled for the implantation of the dental implant. Typically drilling the hole for the dental implant at the correct angle is particularly difficult. Whilst the position may more easily be correctly determined by medical practitioners, judging the correct angle for drilling the hole for the dental implant creates difficulties and is prone to errors in the choice of angle and in holding the angle for the entire time of the drilling. By indicating the drill angle for the whole to be drilled the implantation aid can greatly improve the choice of the correct angle and the constant drilling at the right and, thereby decreasing the risk of mistakes and thus, of subsequent illnesses and/or complications which may in turn lead to further treatments.

In a preferred aspect of the invention, the first aperture and the position for the dental implant are arranged in an overlapping manner. Thus the application of the implantation aid can easily be checked. Furthermore, direct access is provided to the drill site, i.e. the position for the dental implant.

In a further aspect of the invention, the implantation aid comprises a second aperture, wherein the second aperture is adapted to point out a vestibular access point, through which vestibular access point the cavity formations can be resolved. It is particularly difficult for medical practitioners when resolving cavitation formations within the jaw bone after a tooth extraction to determine the direct access point through which the cavitation formations can be resolved. This is due to the fact that the cavitation formations are not visible from the outside, such that finding access point is mostly dependent on the relative position of the cavitation formation inside the jaw bone to structures outside and visible to the medical practitioners. This, therefore, is prone to errors. The second aperture points out a vestibular access point through which vestibular access point the cavitation formations can be resolved, thus reducing a large amount of uncertainty about the position of said access point thereby reducing the risk of errors and consequential illnesses and/or complications resulting therefrom.

In a preferred aspect of the invention, the second aperture is designed such that when the implantation aid is correctly positioned in the region of the jaw, the second aperture and the position of one or more cavitation formations in the region of the jaw are arranged in an overlapping manner. By said arrangement a direct resolution of cavitation formations with a decreased amount of invasive surgery is made possible.

According to a further aspect of the invention, the implantation aid is made at least partially from plastic. This is particular beneficial since plastic is not harmful to the human body. Furthermore, the implantation aid may be manufactured from a plastic which is not so hard as to cause damage when the patient clenches their jaws, such that a strong force is exerted onto the implantation aid and transferred to the teeth. Moreover, damage to the implantation aid in such cases may also be reduced.

In a further aspect of the invention, the implantation aid is at least partially produced using additive manufacturing. Additive manufacturing is efficient and may be formed at a small-scale with ease, such that the implantation aid may even be produced by the medical professionals implanting the dental implant.

According to an aspect of the invention the implantation aid is used for the positioning of a dental implant during the implantation of the dental implant in a region of a jaw and main a further aspect also be used for resolving a cavitation formation in the region of a jaw.

Other features and advantages will become apparent from the following description. The above summary is not meant to limit the scope of the invention.

### BRIEF DESCRIPTION OF THE FIGURES

- Fig. 1A: is an illustrative representation of a radiological image of a region of a jaw taken at a point in time after an extraction of a tooth and after a jaw bone regeneration phase after the extraction.
- Fig. 1B: is another illustrative representation of a radiological image of a region of a jaw taken at a point in time after an extraction of a tooth and before a jaw bone regeneration phase.
- Fig. 2A: is an illustrative representation of a sectional occlusal image of a region of a jaw.
- Fig. 2B: is another illustrative representation of a sectional occlusal image of a region of a jaw.
- Fig. 3A: is an illustrative representation of a sectional buccal image of a region of a jaw.
- Fig. 3B: is an illustrative representation of a sectional lingual image of a region of a jaw.
- Fig. 3C: is an illustrative representation of a sectional mesial image of a region of a jaw.
- Fig. 3D: is an illustrative representation of a sectional distal image of a region of a jaw.
- Fig. 3E: is an illustrative representation of a superposition of two sectional occlusal images of a region of a jaw.
- Fig. 4: is an illustrative representation of a superposition of two sectional buccal/lingual images of a region of a jaw.
- Fig. 5A: is an illustrative representation of an implantation plan.
- Fig. 5B: is an illustrative representation of a file.
- Fig. 6A: is an illustrative representation of a side view of an implantation aid.
- Fig. 6B: is an illustrative representation of a top view of an implantation aid.
- Fig. 6C: is an illustrative representation of an implantation aid fitted to neighbouring structures of an implantation site.

Like elements have same reference numbers.

### DETAILED DESCRIPTION

According to the invention, radiological images are received and analysed in order to determine a position for a dental implant in a region of a jaw in an implantation plan, which implantation plan is provided.

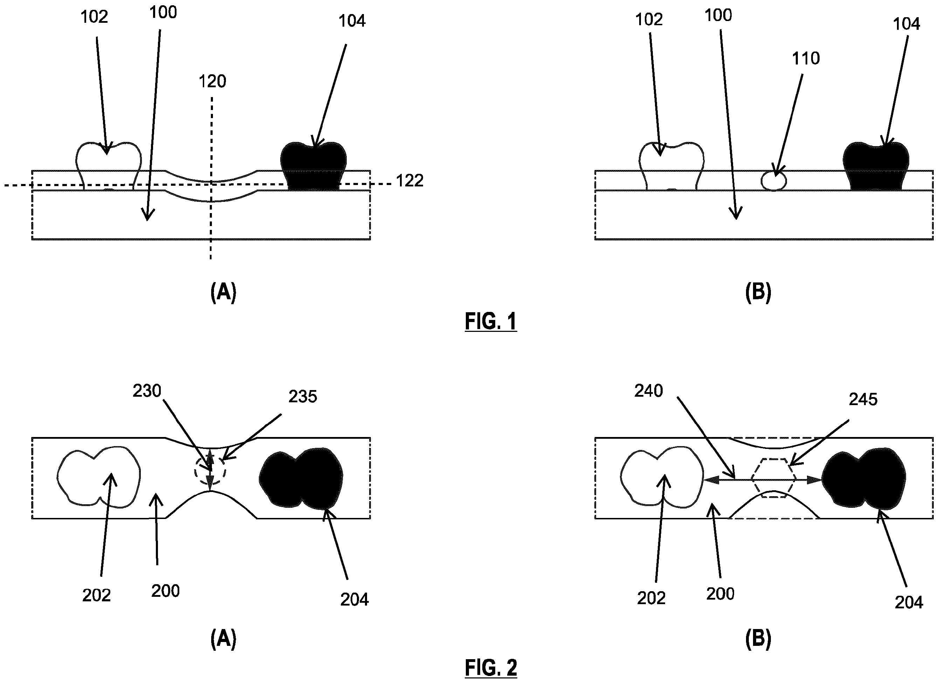

Fig. 1A shows a three-dimensional first radiological image of a region of a jaw. The first radiological image of the region of the jaw may be an extracted part of a radiological image of an entire jaw, cranium or other structure, which radiological image encompasses the region of the jaw. The first radiological image may be rendered as a three-dimensional model. Furthermore, artefacts in the first radiological image, such as noise or projection artefacts etc. may be eliminated through processing of the first radiological image.

In an embodiment of the invention, a first radiological image as illustrated in fig. 1A is received, which first radiological image was taken at a first point in time, which first point in time being after the extraction of a tooth from the region of the jaw (e.g. before or after start of a jaw bone regeneration phase in the region of the jaw). A jaw bone within this disclosure is any bone which houses teeth (e.g. maxilla, mandibula etc.) and is not limited to the mandibula. In the first radiological image of the region of the jaw the jaw bone 100 and neighbouring teeth 102, 104 of the tooth before its extraction are visible. Furthermore, a decreased height of the jaw bone ridge between neighbouring teeth 102, 104 is visible. The first radiological image is a CBCT image of the region of the jaw.

The first radiological image is analysed, such that a position for a dental implant may be determined and provided as part of an implantation plan. Preferably, the analysing includes generating sets of sectional images of the region of the jaw from the first radiological image.

Fig. 2A shows a sectional image, an occlusal view (i.e. looking at the masticatory surface of the teeth) of the region of the jaw of the first radiological image of fig. 1A, which encompasses the jaw bone 200 and neighbouring teeth 202, 204. As is portrayed in fig. 2A, the jaw bone has a reduced bucco-lingual width 230 at the position of the tooth before it extraction between neighbouring teeth 202, 204.

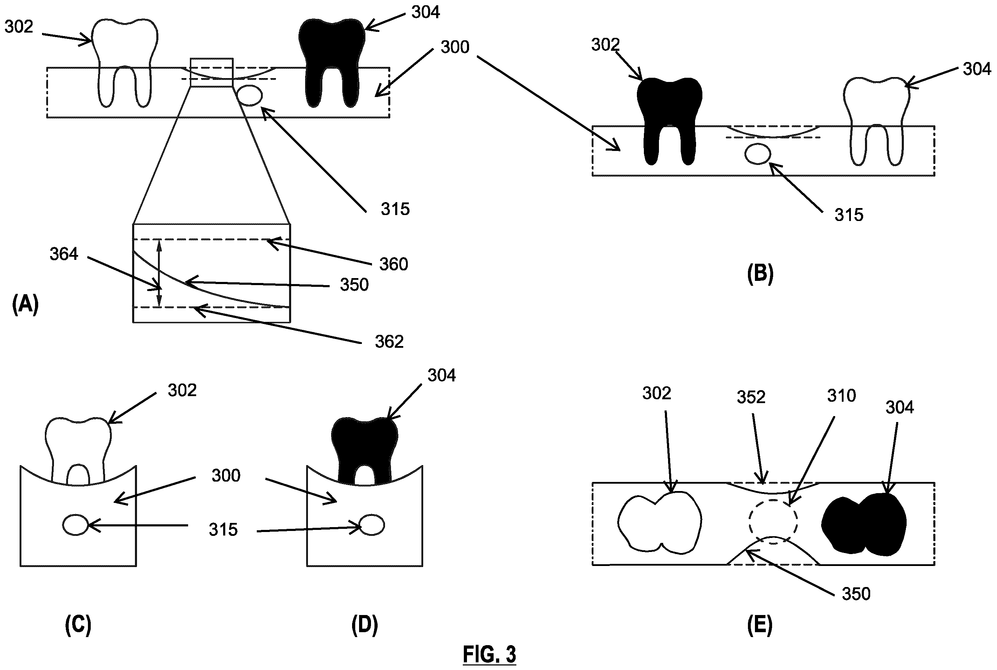

Turning to figs. 3A to D, which all show sectional images of the first radiological image, fig. 3A shows a buccal, fig. 3B a lingual, fig. 3C a mesial and fig. 3D a distal sectional image of the region of the jaw in which the neighbouring teeth 302, 304 and the jaw bone 300 are visible.

Buccal and lingual sectional images are created as sectional images along the dashed line 122 if fig. 1A and parallel to the plane of the illustration. Mesial and distal sectional images are created as sectional images along the dashed line 120 of fig. 1A and orthogonal to the plane of the illustration.

The sectional images are then analysed, wherein the analysing includes identifyinf structural features in the region of the jaw. In this case a structural feature which is identified is cavitation formation 315. Cavitation formation 315 has formed during the healing process of the extraction socket of the tooth after its extraction from the region of the jaw. Based on said identification, the probable position of the tooth before its extraction in he region of the jaw may be determined and provided as part of the implantation plan as the position for the dental implant.

Returning to fig. 2, fig. 2A shows a position 235 for a dental implant as determined by applying the methods in the state of the art, which position being bucco-lingually centrally located on the remaining jaw bone and which position has been determined based solely on the remaining jaw bone itself and the bucco-lingual width thereof. Position 235 for the dental implant is ill suited, because the position 235 does not correspond to the probable position 245 (as shown in fig. 2B) of the tooth before its extraction, such that complications may arise after implantation of the dental implant due to increased mechanical stress applied to the dental implant, which in turn may cause loosening of the dental implant, inflammation, infection or other subsequent complications.

Through the analysis of the first radiological image as described above, the probable position 245 of the tooth before its extraction may be determined, which probable position 245 is provided as the position 245 for the dental implant in the implantation plan. This is shown in fig. 2B. Providing such an implantation plan reduces the risk of subsequent issues with the dental implant such as loosening of the dental implant in the region of the jaw after implantation.

In another embodiment of the invention, the probable position of the tooth before its extraction and thus the position for the dental implant is determined based on an interpolated form of the jaw bone in the region of the jaw. Turning again to fig. 2B, which is an occlusal sectional image of the first radiological image, as shown in fig. 1A, the form of the jaw bone 200 at the first point in time is shown in the continuous line surrounding jaw bone 200. The form of the jaw bone in the vicinity of neighbouring oral structures, here neighbouring teeth 202, 204, is identified and interpolated. The interpolated form is shown by the dashed lines in fig. 2B. Based on the interpolated form of the jaw bone 200, the position 245 of the dental implant, corresponding to the probable position of the tooth before its extraction in the region of the jaw, is determined and provided in the implantation plan for the implantation of the dental implant in the region of the jaw.

In another embodiment of the invention, a first radiological image as illustrated in fig. 1A is received, which first radiological image was taken at a first point in time, which first point in time being after the extraction of a tooth from the region of the jaw and which first point in time being after a jaw bone regeneration phase. In the first radiological image of the region of the jaw the jaw bone 100 and neighbouring teeth 102, 104 of the tooth before its extraction are visible. Furthermore, a decreased height of the jawbone ridge between neighbouring teeth 102, 104 is visible. Additionally, a second radiological image as illustrated in fig. 1B is received, which second radiological image was taken at a second point in time, which second point in time being after the extraction of a tooth from the region of the jaw and which second point in time being before a jaw bone regeneration phase. In the second radiological image of the region of the jaw the jaw bone 100, neighbouring teeth 102, 104 and the extraction socket 110 of the extracted tooth are visible. The height of the jaw bone ridge is not decreased compared to the height of the jaw bone ridge in the vicinity of the neighbouring teeth 102, 104 in the second received radiological image.

Preferably, the received radiological images are computer tomography images of the region of the jaw or derived from computed tomography images, such as three-dimensional models generated on the basis of computed tomography images. It is further preferred that the received radiological images are denoised and have been processed to reduce artefacts.

The received radiological images are analysed, such that a position for a dental implant may be determined and provided as part of an implantation plan. Preferably, the analysing includes generating sets of sectional images of the region of the jaw from the received radiological images, especially of the first and second radiological images. The sectional images in every set of sectional images are superimposed, such that the determination of the probable position of the tooth before its extraction is facilitated.

Fig. 2A shows a sectional image, an occlusal view (i.e. looking at the masticatory surface of the teeth) of the region of the jaw of the first radiological image of fig. 1A, which encompasses the jaw bone 200 and neighbouring teeth 202, 204. As is portrayed in fig. 2A, the jaw bone has a reduced bucco-lingual width 230 at the position of the tooth before it extraction between neighbouring teeth 202, 204.

A corresponding sectional image is generated of the second radiological image as part of the set of sectional images.

Further sectional images may be generated in other directions. Fig. 3A shows a sectional buccal image of the first radiological image of the region of the jaw containing the jaw bone 300, neighbouring structures of the tooth before its extraction, i.e. neighbouring teeth 302, 304 and further features. Fig. 3B shows the corresponding sectional lingual image. Buccal and lingual sectional images are created as sectional images along the dashed line 122 and parallel to the plane of the illustration. Figs. 3C and 3D show corresponding sectional mesial and distal images respectively. Mesial and distal sectional images are created as sectional images along the dashed line 120 and orthogonal to the plane of the illustration.

Fig. 3E shows an exemplary superposition of the occlusal sectional images of the set of sectional images containing occlusal sectional images of the first and second radiological images. Since the second radiological image was taken after the extraction of the tooth and before a bone regeneration phase, the extraction socket 310 is visible in the super imposed image. Furthermore, jaw bone form 352 as it was at the second point in time, at which the second radiological image was taken is also visible. In the jaw bone regeneration phase the extraction socket 310 has healed (at least superficially), such that it is no longer visible in the sectional image of the first radiological image. The jaw bone form 350 has undergone structural changes (e.g. due to jaw bone atrophy) such that in the superimposed image of fig. 3E this is visible.

By said analysis of the received radiological images, especially the first and second radiological images, a probable position of the tooth before its extraction is determined and provided in an implantation plan for the implantation of the dental implant. Returning to fig. 2, fig. 2A shows a position 235 for a dental implant as determined by applying the methods in the state of the art, which position being centrally located on the remaining jaw bone. Position 235 for the dental implant is ill suited, because the position 235 does not correspond to the probable position 245 (as shown in fig. 2B) of the tooth before its extraction, such that complications may arise after implantation of the dental implant due to increased mechanical stress applied to the dental implant, which in turn may cause loosening of the dental implant, inflammation, infection or other subsequent complications.

Through the analysis of the received radiological images as described above, the probable position 245 of the tooth before its extraction may be determined, which probable position 245 is provided as the position 245 for the dental implant in the implantation plan. This is shown in fig. 2B. Providing such an implantation plan reduces the risk of subsequent issues with the dental implant such as loosening of the dental implant in the region of the jaw after implantation.

Preferably, during the analysis of the radiological images, structural changes in the region of the jaw such as jaw bone atrophy, are identified in the sectional images of the first radiological image (i.e. independently of the second radiological image, such that the identification may also occur in embodiments of the invention, in which only a first radiological image is received).It is particularly preferred, that the change is a change in height of the jawbone ridge in the region of the jaw. The change in height of the jaw bone ridge may be determined in the buccal or lingual sectional images. As illustrated in fig. 3A, the jaw ridge height may be determined in the buccal sectional image of the first radiological image. This is achieved by defining a reference line 360 which corresponds to the height of the jaw ridge before the extraction of the tooth. Preferably, this reference line 360 is a mesio-distal bone line, which is drawn at the jaw ridge height of the neighbouring oral structures, in this example the neighbouring teeth 302, 304. The actual height of the jaw ridge is determined by determining a tangent 362 to the lowest point of the jaw ridge, which tangent 362 being parallel to the reference line 360. It is clear to the skilled person that the tangent 362 does not need to be a tangent in the strict mathematical sense, that it lies at a right angle to the radius of the jaw ridge at its lowest point, but that it touches the lowest point of the jaw ridge and is parallel to the reference line 360. The distance 364 between the two lines 360, 362 is determined, which distance 364 corresponds to the change in height of the jaw ridge. When the sectional images of the first and second radiological images are superimposed, other methods for the determination of the change in jaw ridge height are equally possible. For example, the average height, maximal height or lowest height of the jaw ridge at the position of the tooth before its extraction may be used as a reference line.

The determined distance 364 may be used in the determination of an implant type to be used for the implantation according to the implantation plan. It is beneficial for said determined implant type to be provided in the implantation plan. For example, when the distance is less than but greater than 1 mm, a tissue level implant may be indicated in the implantation plan, whilst when the distance is less than 1 mm a bone level implant may be indicated in the implantation plan.

Preferably, the analysing of the received radiological images, especially the first radiological image, comprises the determination of a mesio-distal distance between neighbouring oral structures (i.e. possibly independently of the second radiological image, such that the determination may also occur in embodiments of the invention, in which only a first radiological image is received). Fig. 2B shows the mesio-distal distance between the neighbouring teeth 302, 304 in the sectional occlusal image of the first radiological image. However, it is understood that the mesio-distal distance may be determined in any number of ways, especially in the sectional buccal/lingual images of the first radiological image. Fig. 2A shows the determination of the bucco-lingual width 230 of the jaw ridge in the first radiological image, which in the state of the art is used for the determination of the diameter of the dental implant to be implanted. Fig. 2B shows the mesio-distal distance which in the embodiment of the invention is used instead. The use of the mesio-distal distance for said determination allows a better matching of the dental implant to the tooth before its extraction and the neighbouring structures, such that the dental implant more closely emulates the extracted tooth and its biomechanical properties and/or increases the stability of the dental implant. The determined diameter of the dental implant may be provided in the implantation plan.

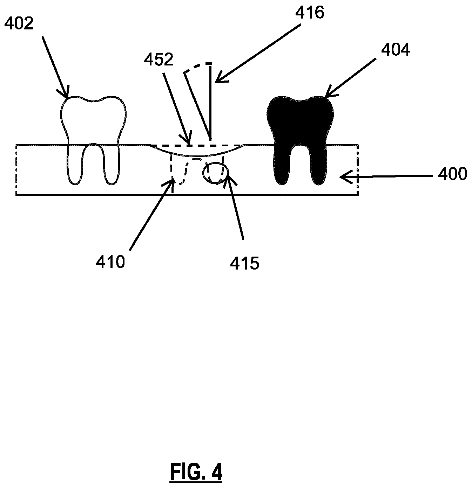

Furthermore, a drill angle 416 for the hole to be drilled for the dental implant to be implanted may be determined. The determined drill angle 416 may be provided in the implantation plan. The drill angle 416 may be determined from the sectional images, especially of the first radiological image (i.e. possibly independently of the second radiological image, such that the determination of the drill angle 416 may also occur in embodiments of the invention, in which only a first radiological image is received) and/or the second radiological image, or the superposition of the sectional images. The drill angle 416 may be chosen such that it closely resembles the angle of the extraction socket, which corresponds to the angle of the root of the tooth before its extraction. Fig. 4 shows a superposition of two sectional buccal images of the region of the jaw. The extraction socket 410 is visible, such that the drill angle 416 for the hole may be determined. Other features may be taken account of, as will be described in the following.

More preferably, the analysing includes identifying structural features in the region of the jaw, such as cavitation formations. The cavitation formations may not be visible in the radiological images as such. Thus, the analysis may include said identification on the sectional images, preferably on the sectional images of the first radiological image. Figs. 3A-D show the buccal, lingual, mesial and distal sectional images of the first radiological image shown in fig. 1A. The first radiological image was taken at a first point in time, which first point in time being after the extraction of the tooth in the region of the jaw and after a jaw bone regeneration phase, which jaw bone regeneration phase is after the extraction of the tooth. During the jaw bone regeneration phase cavitation formations may develop, such as cavitation formation 315 in figs. 3A-D. Strucural changes in the region of the bone leading to the cavitation formations 315 may also be identified. In order for structural changes in the area of the cavitation formations 315 to be identified, the neighbouring structures, such as neighbouring teeth 302, 304 and endemic bone may be identified in the sectional images of the first and second radiological images. The extraction socket 110, 310, 410 is identified in the sectional images of the second radiological image and the cavitation formations are identified as described above in the sectional images of the first radiological image. Superimposing said sectional images allows the analysis of the cavitation formation and the determination of the amount of healing of the extraction socket 110, 310, 410, e.g. less than 95%, 90% or 80% or of the socket 110, 310, 410 has fully healed.

Other values are also identifiable. The determined amount of healing may be provided in the implantation plan.

The identification of structural features in the region of the jaw, such as cavitation formations 315 may also be relevant for the determination of the drill angle 416, such that the drill angle 416 may be determined such that the hole to be drilled does not intersect, or fully intersects existing cavitation formations 315.

Preferably, it is determined during the generation of the implantation plan, whether the implantation of the dental implant requires jawbone augmentation prior to and/or during the implantation procedure. For example, the position 245 for the dental implant indicating the probable position of the tooth before its extraction in fig. 2B extends out of the remaining jaw bone, such that an implantation without jaw bone augmentation is not possible. The diameter of the dental implant is determined by the mesio-distal distance between the neighbouring teeth, such that the bucco-lingual width 230 of the jaw ridge is not taken into account, thus necessitating the augmentation. Typically available dental implants have diameters between 3 mm and 5 mm. For example, should the bucco-lingual width 230 of the jaw ridge exceed 5.5 mm it may be determined that bone augmentation is required during the implantation. The amount of augmentation may also be determined. The amount may be determined based on the diameter of the implant, when the bucco-lingual width 230 of the jaw ridge is not large enough to accommodate the dental implant, or may be determined in a general manner, when the implant can be accommodated, but the augmentation is required for other reasons, such as bone health, stability etc. In the same example, it may be determined that when the bucco-lingual width 230 of the jaw ridge is less than 5.5 mm, augmentation prior to the implantation is required. In such cases a redetermination can occur after the augmentation of the bone. It is beneficial for the described determinations to be provided within the implantation plan.

It is further preferred that it is determined during the generation of the implantation plan, whether existing cavitation formations must be resolved prior to and/or during the implantation procedure. For example, cavitation formation 415 in fig. 4 intersects the area in which the dental implant is to be implanted. Furthermore, cavitation formation 415 extends beyond the area, such that the dental implant may not sit firmly in the jaw bone and may be prone to loosening. Thus, it may be determined that the cavitation formation 415 must be resolved prior to the implantation of a dental implant at the position 245 for the dental implant.

The determinations regarding the jaw bone augmentation and/or cavitation formation resolution are based on the analysed radiological images, preferably the first radiological image (i.e. possibly independently of the second radiological image, such that the determination may also occur in embodiments of the invention, in which only a first radiological image is received) and/or the second radiological image.

It is further preferred that the position 245 for the dental implant is provided as a set of three-dimensional coordinates, the coordinates being in a reference coordinate system of one of the received radiological images, preferably in the reference coordinate system of the first radiological image. By the provision of such a set of coordinates easy visualization of the position 245 for the dental implant is facilitated, thereby easing the implantation procedure for medical practitioners.

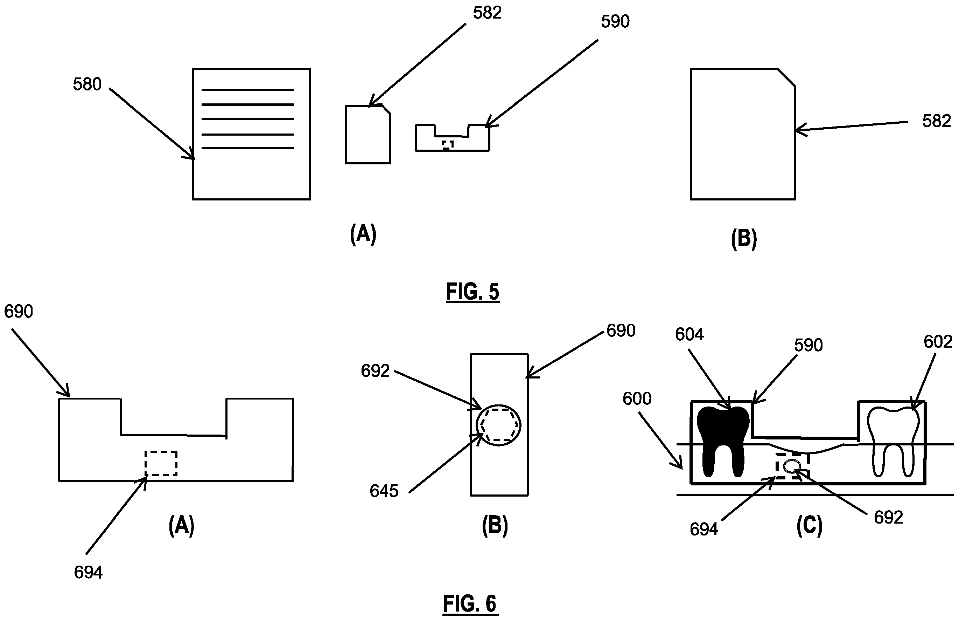

Preferably, a file containing mesh coordinates of a three-dimensional model of the dental implant is generated and preferably also provided as part of the implantation plan. The file may be used for visualization purposes in any one of the radiological images, especially in combination with the set of three-dimensional coordinates. Moreover, the file is adapted to be used in the additive manufacturing of an implantation aid, which implantation aid may be used to facilitate the implantation of the dental implant according to the implantation plan. Fig. 5 shows an implantation plan 580 entailing information specifying details described above with regard to embodiments of the invention (such as the diameter of the dental implant, the position 245 for the dental implant etc.). The implantation plan may further comprise a file 582 containing the three-dimensional mesh. Additionally the implantation plan may comprise an implantation aid 590 according to the invention.

Both the file containing the three-dimensional model of the dental implant and the three-dimensional coordinates may also be provided in embodiments of the invention, in which only the first radiological image is received or analysed.

In an embodiment of the invention the implantation plan 580 is used for the implantation of a dental implant at the position 245 indicated in the implantation plan.

In another embodiment, the file 583 is used in the manufacturing of an implantation aid 590, for the implantation of a dental implant at the position 245 indicated in the implantation plan 580.

Fig. 6 shows an implantation aid 690 according to an embodiment of the invention. The implantation aid is configured to be placed adjacent, in this case onto oral structures, in this case the gum/jaw 600 and neighbouring teeth 602, 604, and assists in the positioning of the dental implant. Fig. 6A shows a side view of the implantation aid 690. Fig. 6B shows a top view of the implantation aid. The implantation aid comprises a first aperture 692, which indicates the position 645 for the dental implant. The first aperture 692 is also configured to indicate a drill angle 416 for a hole to be drilled for the implantation of the dental implant. The angle may be indicated by any suitable means. In the embodiment of fig. 6, the angle 416 is indicated by the first aperture 692, which may be configured as a guiding mechanism, by being configured as a slanted tube, which guides the drill head. It may also be envisaged, that the position 645 is indicated by a visual cue, which visual cue is visible through the first aperture 692 only at the drill angle 416. Other means may also be implemented. The implantation aid further comprises a second aperture 694, which second aperture 694 indicates a vestibular access point for the resolution of cavitation formation 615. As shown in fig. 6C, the implantation aid is placed on the neighbouring theeth 602, 604, which provide a stabilization to the implantation aid. Thus, the hole for the dental implant may be drilled, at the position 645 for the dental implant, without the implantation aid 690 being displaced. This allows for a precise placement of the dental implant. The implantation aid 690 is made from plastic, such that it poses no health risks to the patient. It may further be made from a transparent material, preferably a transparent plastic, such that the neighbourng structures (gere neighbouring teeth 602, 604) and the vicinity of the position 645 for the dental implant remain visible during the implantation procedure.

Various aspects of the present invention may be used alone, in combination, or in a variety of arrangements not specifically discussed in the embodiments described in the foregoing and is therefore not limited in its application to the details and arrangement of components set forth in the foregoing description or illustrated in the figures. For example, aspects described in one embodiment may be combined in any manner with aspects described in other embodiments.

Also, the invention may be embodied as a method, of which an example has been provided. The acts performed as part of the method may be ordered in any suitable way. Accordingly, embodiments may be constructed in which acts are performed in an order different than illustrated, which may include performing some acts simultaneously, even though shown as sequential acts in illustrative embodiments.

Use of ordinal terms such as "first," "second," "third," etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements. Also, the phraseology and terminology used herein is for the purpose of description and should not be regarded as limiting. The use of "including," "comprising," or "having," "containing," "involving," and variations thereof herein, is meant to encompass the items listed thereafter and equivalents thereof as well as additional items.

Further, though advantages of the present invention are indicated, it should be appreciated that not every embodiment of the invention described herein will include every described advantage. Some aspects and embodiments may not implement any features described as advantageous herein and in some instances one or more of the described features may be implemented to achieve further embodiments. Accordingly, this description and these figures are by way of example only. The invention is defined by the appended claims.

The present invention can (alternatively) be described by one or more of the following numbered aspects:

### ASPECTS

1. A method of planning an implantation of a dental implant, comprising:
   receiving one or more radiological images of a region of a jaw, the received radiological images comprising at least a first radiological image of the region of the jaw in which region a tooth extraction socket is located, wherein the first radiological image was taken at a first point in time, which first point in time being after an extraction of a tooth from the region of the jaw;
   analysing the received one or more radiological images including analysing the first radiological image;
   generating an implantation plan for the implantation of the dental implant in the region of the jaw based on the analysed radiological images, the generating comprising:
      determining a position for the dental implant in the region of the jaw, which position indicates a probable position of the tooth before its extraction in the region of the jaw; and
      indicating the determined position in the implantation plan; and
   providing the generated implantation plan.
2. The method of aspect 1, wherein the first point in time is after a period of time for a partial or complete jaw bone regeneration phase in the region of the jaw after the extraction of the tooth.
3. The method of aspect 1, wherein the first point in time is before a start of jaw bone regeneration phase in the region of the jaw, such as directly after the extraction of the tooth in the region of the jaw, preferably less than one week after the extraction, more preferably less than twenty four hours after the extraction.
4. The method of aspect 1 or 2, wherein the received radiological images further comprise a second radiological image of the region of the jaw, wherein the second radiological image was taken at a second point in time, which second point in time being before the first point in time and preferably before the start of the period of time for the partial or complete jaw bone regeneration phase.
5. The method of aspect 3, wherein the received radiological images further comprise a second radiological image of the region of the jaw, wherein the second radiological image was taken at a third point in time, which third point in time being after the first point in time and preferably after a period of time for the partial or complete jaw bone regeneration phase in the region of the jaw after the extraction of the tooth.
6. The method of aspect 4 or 5, wherein the first point in time and the second or third point in time are separated by at least four weeks.
7. The method of any one of the preceding aspects, wherein the received radiological images are three-dimensional computed tomography images, preferably CBCT-images, more preferably denoised CBCT-images.
8. The method of any one of the preceding aspects, wherein the analysing of the one or more received radiological images further comprises:
   generating sets of sectional images of the region of the jaw from the one or more received radiological images, wherein every set contains at least one sectional image of the first radiological image, and preferably of the second radiological image,
   wherein the sectional images in every set correspond to one another, and
   wherein the sectional images in every set preferably have the same sectional plane and orientation.
9. The method of aspect 8, the analysing of the one or more received radiological images further comprising:
   superimposing the sectional images of every set of sectional images.
10. The method of any one of the preceding aspects, wherein the analysing of the one or more radiological images further comprises:
   identifying, in the analyzed one or more images, structural features and/or structural changes in the region of the jaw, particularly neighbouring structures of the tooth before its extraction from the region of the jaw, preferably neighbouring teeth, an extraction socket, a form of the jaw bone, cavitation formations and/or atrophies in the region of the jaw.
11. The method of aspect 10, wherein the identifying of structural features and/or structural changes is done based on the sets of sectional images.
12. The method of aspect 10 or 11, wherein the sets each contain sectional images of the second radiological image and wherein the identifying is preferably done based on the superimposed sectional images of the first and second radiological images.
13. The method of any one of aspects 10 to 12, wherein the determining of the position for the dental implant in the region of the jaw is done based on the identified structural features and/or structural changes in the region of the jaw, preferably identified cavitation formations and/or atrophies in the region of the jaw.
14. The method of any one of aspects 10 to 13, wherein the identified structural changes comprise changes in a height and/or a width of a jaw ridge of the jaw in the region of the jaw.
15. The method of any one of aspects 8 to 14, wherein the generating of sets of sectional images further comprises:
   generating sectional images of the region of the jaw in occlusal, buccal, lingual, mesial and/or distal direction.
16. The method of aspect 14 or 15, wherein the identifying of the change of a height of a jaw ridge is done on the basis of a reference line, which reference line preferably being a mesio-distal bone line in the sectional images of one of the received radiological images, wherein the mesio-distal bone line is a line connecting the jaw ridge at neighbouring oral structures of the tooth before its extraction in the region of the jaw, particularly between neighbouring teeth.
17. The method of aspect 16, wherein the identifying of the change of a height of a jaw ridge further comprises:
   determining an actual height of a jaw ridge in the region of the jaw by determining a tangent to the jaw ridge at the lowest point of the jaw ridge in the first radiological image and/or the sectional images of the first radiological image, the tangent being parallel to the reference line; and
   determining the distance between the tangent and the reference line.
18. The method of aspect 17, wherein the generating of the implantation plan further comprises:
   determining an implant type for the dental implant based on the distance between the tangent and the reference line.
19. The method of any one of the preceding aspects, the analysing of the one or more radiological images further comprising:
   determining a mesio-distal distance between neighbouring oral structures of the tooth, particularly between neighbouring teeth in at least one of the received radiological images, preferably in the first radiological image and/or the sectional images of the first radiological image.
20. The method of any one of the preceding aspects, wherein the analysing of the one or more radiological images further comprises:
   determining a drill angle for a hole to be drilled for the implantation of the dental implant.
21. The method of aspect 19, wherein the generating of the implantation plan further comprises:
   determining a diameter of the dental implant based on the mesio-distal distance.
22. The method of any one of the preceding aspects, wherein the determined position for the dental implant in the region of the jaw is determined as a set of three-dimensional coordinates in a reference coordinate system of one of the received radiological images.
23. The method of any one of the preceding aspects, wherein the generating of the implantation plan further comprises:
   generating a file containing mesh coordinates of a three-dimensional data model of the dental implant, wherein the file is adapted to be used for additive manufacturing of an implantation aid according to any one of aspects 50 to 57.
24. The method of any one of the preceding aspects, wherein the generating of the implantation plan further comprises:
   determining whether the implantation of the dental implant in the region of the jaw requires bone augmentation in the region of the jaw prior to and/or during the implantation.
25. The method of any one of the preceding aspects, wherein the generating of the implantation plan further comprises:
   determining how much bone augmentation in the region of the jaw is required prior to and/or during the implantation.
26. The method of aspect 10, wherein the generating of the implantation plan further comprises:
   determining whether existing cavitation formations in the region of the jaw must be resolved before and/or during the implantation of the dental implant.
27. The method of any one of aspects 18 to 26, wherein the providing of the implantation plan comprises:
   providing in the implantation plan information, which information specifies the determined type of dental implant.
28. The method of any one of aspects 24 to 27, wherein the providing of the implantation plan comprises:
   providing in the implantation plan information, which information specifies whether the implantation of the dental implant in the region of the jaw requires bone augmentation in the region of the jaw prior to the implantation.
29. The method of any one of aspects 25 to 28, wherein the providing of the implantation plan comprises:
   providing in the implantation plan information, which information specifies how much bone augmentation in the region of the jaw is required prior to the implantation.
30. The method of any one of aspects 21 to 29, wherein the providing of the implantation plan comprises:
   providing in the implantation plan the determined diameter of the dental implant.
31. The method of any one of aspects 22 to 30, wherein the providing of the implantation plan comprises:
   providing in the implantation plan the set of three-dimensional coordinates.
32. The method of any one of aspects 23 to 31, wherein the providing of the implantation plan comprises:
   providing in the implantation plan the file.
33. The method of any one of aspects 26 to 32, wherein the providing of the implantation plan comprises:
   providing in the implantation plan information, which information specifies whether existing cavitation formations in the region of the jaw must be resolved before and/or during the implantation of the dental implant.
34. The method of any one of aspects 20 to 33, wherein the providing of the implantation plan comprises:
   providing in the implantation plan the determined drill angle.
35. The method of any one of the preceding aspects, wherein the providing of the implantation plan comprises:
   providing an implantation aid according to any one of aspects 50 to 57.
36. The method of any one of the preceding aspects, wherein the analysing includes:
   identifying a form of the jaw bone in the vicinity of neighbouring oral structures of the tooth before its extraction in the region of the jaw; and
   interpolating the identified form; and
   wherein the determining of the position for the dental implant in the region of the jaw is done based on the interpolated form.
37. The method of any one of the preceding aspects, wherein the analysing of the one or more received radiological images is performed by a computer.
38. The method of any one of aspects 1 to 37, wherein all operations of the method, except for the providing in the implantation plan of the implantation aid, are performed by a computer.
39. The method of aspects 37 or 38, wherein the operations performed by the computer are performed automatically.
40. The method of any one of the preceding aspects, wherein the method does not include an insertion of any component, such as the dental implant, into a mouth of a patient, in particular does the method not include any implanting of the dental implant.
41. The method of any one of the preceding aspects, wherein the method does not involve presence of a patient having the jaw.
42. The method of any one of the preceding aspects, wherein the method does not include performing any bone augmentation and/or cavitation formation resolution in the region of the jaw.
43. Storage medium comprising commands, which commands when carried out by a processor, cause the processor to perform the method of any one of aspects 1 to 42.
44. Computer programme comprising commands, which commands when carried out by a processor, cause the processor to perform the method of any one of aspects 1 to 42.
45. Computing device or system adapted to perform the method of any one of aspects 1 to 42.
46. Implantation plan for the implantation of a dental implant in a region of a jaw, which implantation plan is obtainable by the method of any one of aspects 1 to 42, wherein the implantation plan entails a position, preferably three-dimensional coordinates, for the dental implant in a region of the jaw, which position indicates the probable position of a tooth before its extraction in the region of the jaw.
47. Use of the implantation plan of aspect 46 for the implantation of a dental implant in a region of a jaw.
48. A file, preferably an .STL file, which file is obtainable by the method of aspect 23, wherein the file contains mesh coordinates of a three-dimensional data model of the dental implant to be implanted in a region of a jaw, wherein the file is adapted to be used for additive manufacturing of an implantation aid according to any one of aspects 50 to 57.
49. Use of a file according to aspect 48 for additive manufacturing of an implantation aid according to any one of aspects 50 to 57.
50. An implantation aid configured to be placed adjacent to an oral structure, preferably a tooth, to assist in positioning a dental implant in a region of a jaw at a position for the dental implant.
51. The implantation aid according to aspect 50, wherein the implantation aid is configured to be placed on the structures, particularly teeth, surrounding the position for the dental implant.
52. The implantation aid according to aspect 50 or 51, wherein the implantation aid comprises a first aperture, the first aperture being designed such that when the implantation aid is correctly positioned in the region of the jaw, the first aperture indicates a drill angle for a hole to be drilled for the implantation of the dental implant.
53. The implantation aid according to aspect 52, wherein the first aperture and the position for the dental implant are arranged in an overlapping manner.
54. The implantation aid according to any one of aspects 50 to 53, wherein the implantation aid comprises a second aperture, wherein the second aperture is adapted to point out a vestibular access point, through which vestibular access point the cavitation formations can be resolved.
55. The implantation aid according to aspect 54, the second aperture being designed such that when the implantation aid is correctly positioned in the region of the jaw, the second aperture and the position of one or more cavitation formations in the region of the jaw are arranged in an overlapping manner.
56. The implantation aid according to any one of aspects 50 to 55, wherein the implantation aid is made at least partially from plastic.
57. The implantation aid according to any one of aspects 50 to 56, wherein the implantation aid is at least partially produced using additive manufacturing.
58. Use of an implantation aid according to any one of aspects 50 to 57 for the positioning of a dental implant during the implantation of the dental implant in a region of a jaw.
59. Use of an implantation aid according to any one of aspects 50 to 57 for resolving a cavitation formation in a region of a jaw.

## Claims

1. A method of planning an implantation of a dental implant, comprising:
receiving one or more radiological images of a region of a jaw, the received radiological images comprising at least a first radiological image of the region of the jaw in which region a tooth extraction socket (110, 310, 410) is located, wherein the first radiological image was taken at a first point in time, which first point in time being after an extraction of a tooth from the region of the jaw;
analysing the received one or more radiological images, including analysing the first radiological image;
generating an implantation plan (580) for the implantation of the dental implant in the region of the jaw based on the analysed radiological images, the generating comprising:
determining a position (245, 645) for the dental implant in the region of the jaw, which position indicates a probable position of the tooth before its extraction in the region of the jaw; and
indicating the determined position (245, 645) in the implantation plan (580);
and
providing the generated implantation plan (580).

2. The method of claim 1, wherein the first point in time is after a period of time for a partial or complete jaw bone regeneration phase in the region of the jaw after the extraction of the tooth.

3. The method of claim 1 or 2, wherein the received radiological images further comprise a second radiological image of the region of the jaw, wherein the second radiological image was taken at a second point in time, which second point in time being before the first point in time and preferably before the start of the period of time for the partial or complete jaw bone regeneration phase.

4. The method of any one of the preceding claims, wherein the received radiological images are three-dimensional images, preferably three-dimensional computed tomography images preferably CBCT-images, more preferably denoised CBCT-images; and
wherein the analysing of the one or more received radiological images further comprises:
generating sets of sectional images of the region of the jaw from the one or more received radiological images, wherein every set contains at least one sectional image of the first radiological image, and preferably of the second radiological image,
wherein the sectional images in every set correspond to one another, and
wherein the sectional images in every set preferably have the same sectional plane and orientation; and
preferably, superimposing the sectional images of every set of sectional images.

5. The method of any one of the preceding claims, wherein the analysing of the one or radiological images further comprises:
identifying, in the analyzed one or more images, structural features and/or structural changes in the region of the jaw, particularly neighbouring structures of the tooth before its extraction from the region of the jaw, preferably neighbouring teeth (102, 104, 202, 204, 302, 304, 402, 404, 602, 604), an extraction socket (110, 310, 410), a form (352) of the jaw bone (100, 200, 300, 400, 600), cavitation formations (315, 415, 692) and/or atrophies in the region of the jaw.

6. The method of claim 5, wherein the identifying of structural features and/or structural changes is done based on the sets of sectional images, preferably wherein the sets each contain sectional images of the second radiological image and wherein the identifying is preferably done based on the superimposed sectional images of the first and second radiological images.

7. The method of claim 5 or 6, wherein the determining of the position (245, 645) for the dental implant in the region of the jaw is done based on the identified structural features and/or structural changes in the region of the jaw, preferably identified cavitation formations (315, 415, 692) and/or atrophies in the region of the jaw.

8. The method of any one of claims 5 to 7, wherein the identified structural changes comprise changes in a height and/or a width of a jaw ridge of the jaw in the region of the jaw, wherein the identifying of the change of a height of a jaw ridge is done on the basis of a reference line (360), which reference line preferably being a mesio-distal bone line (360) in the sectional images of one of the received radiological images, wherein the mesio-distal bone line (360) is a line connecting the jaw ridge at neighbouring oral structures of the tooth before its extraction in the region of the jaw, particularly between neighbouring teeth (102, 104, 202, 204, 302, 304, 402, 404, 602, 604);
wherein the identifying of the change of a height of a jaw ridge further comprises:
determining an actual height of a jaw ridge in the region of the jaw by determining a tangent (362) to the jaw ridge at the lowest point of the jaw ridge in the first radiological image and/or the sectional images of the first radiological image, the tangent (362) being parallel to the reference line (360); and
determining the distance (364) between the tangent (362) and the reference line (360);
wherein the generating of the implantation plan (580) further comprises:
determining an implant type for the dental implant based on the distance (364) between the tangent (362) and the reference line (360); and
wherein the providing of the implantation plan (580) comprises:
providing in the implantation plan (580) information, which information specifies the determined type of dental implant.

9. The method of any one of the preceding claims, the analysing of the one or more radiological images further comprising:
determining a mesio-distal distance (240) between neighbouring oral structures of the tooth, particularly between neighbouring teeth (102, 104, 202, 204, 302, 304, 402, 404, 602, 604) in at least one of the received radiological images, preferably in the first radiological image and/or the sectional images of the first radiological image;
wherein the generating of the implantation plan (580) further comprises:
determining a diameter of the dental implant based on the mesio-distal distance (240); and
wherein the providing of the implantation plan (580) comprises:
providing in the implantation plan (580) the determined diameter of the dental implant.

10. The method of any one of the preceding claims, wherein the analysing of the one or more radiological images further comprises:
determining a drill angle (416) for a hole to be drilled for the implantation of the dental implant; and
wherein the providing of the implantation plan (580) comprises:
providing in the implantation plan (580) the determined drill angle (416).

11. The method of any one of the preceding claims, wherein the generating of the implantation plan (580) further comprises:
determining how much bone augmentation in the region of the jaw is required prior to and/or during the implantation; and
wherein the providing of the implantation plan (580) comprises:
providing in the implantation plan (580) information, which information specifies how much bone augmentation in the region of the jaw is required prior to the implantation.

12. The method of any of the preceding claims, wherein the generating of the implantation plan (580) further comprises:
determining whether existing cavitation formations (315, 415, 692) in the region of the jaw must be resolved before and/or during the implantation of the dental implant; and
wherein the providing of the implantation plan (580) comprises:
providing in the implantation plan (580) information, which information specifies whether existing cavitation formations (315, 415, 692) in the region of the jaw must be resolved before and/or during the implantation of the dental implant.

13. Storage medium comprising commands, which commands when carried out by a processor, cause the processor to perform the method of any one of the preceding claims.

14. Computer programme comprising commands, which commands when carried out by a processor, cause the processor to perform the method of any one of claims 1 to 12.

15. Computing device or system adapted to perform the method of any one of claims 1 to 12.

16. Implantation plan (580) for the implantation of a dental implant in a region of a jaw, which implantation plan (580) is obtainable by the method of any one of claims 1 to 12., wherein the implantation plan (580) entails a position (245, 645), preferably three-dimensional coordinates, for the dental implant in a region of the jaw, which position (245, 645) indicates the probable position of a tooth before its extraction in the region of the jaw.
